# EUROPEAN PATENT APPLICATION

(11) **EP 4 335 495 A1**
(43) Date of publication of application: **13.03.2024**
(21) Application number: 22799137.9
(22) Date of filing: 04.05.2022
(51) Int. Cl.: A61N 7/02, B06B 1/06, A61N 7/00

(54) **HIGH INTENSITY FOCUSED ULTRASOUND-BASED ULTRASOUND PROBE AND DEVICE FOR SKIN TREATMENT**

(30) Priority: 06.05.2021 KR 20210058823; 20.04.2022 KR 20220048838
(71) Applicant: VIOL Co. Ltd., Bundang-gu Seongnam-si, Gyeonggi-do, 13510 (KR)
(72) Inventor: CHANG, Jinho, Seoul 07997 (KR); KIM, Jin Woo, Seoul 04096 (KR); KIM, Ju Hwan, Gwangmyeong-si Gyeonggi-do 14318 (KR); NA, Jong Ju, Seoul 05647 (KR); LEE, Dukkyu, Seoul 04947 (KR); KIM, Joungpil, Hwaseong-si Gyeonggi-do 18322 (KR)
(74) Representative: Maiwald GmbH
(86) International application number: PCT/KR2022/006438
(87) International publication number: WO 2022/235094

(57) **Abstract**

A high intensity focused ultrasound (HIFU) probe according to an embodiment of the present invention includes: a transducer module including a plurality of transducer elements generating ultrasound by receiving electrical signals; and a circuit providing a driving signal to the plurality of transducer elements based on a control signal received from a control system, in which the plurality of transducer elements having a concave shape in a first axial direction, which is a focusing direction of ultrasound, are arranged in an array in a second axial direction perpendicular to a first axis with a kerf interposed therebetween, and at least two different elements of the plurality of transducer elements are configured to emit high intensity focused ultrasound at different times based on the driving signal.

## Description

### [Technical Field]

The present disclosure relates to a high intensity focused ultrasound (HIFU) probe and an ultrasound device for skin treatment employing the probe, and more particularly, to a high intensity focused ultrasound probe and an ultrasound device capable of focusing high intensity ultrasound for skin treatment on a plurality of spots and at a plurality of depths without mechanical movement of a transducer element based on a geometrical structure and arrangement of an array type transducer element.

### [Background Art]

Recently, the use of high intensity focused ultrasound (HIFU) for skin treatments such as skin lifting and skin tightening is attracting much attention.

The high intensity focused ultrasound is a technology for treating lesions by focusing high intensity acoustic energy on a local site in the body to increase the temperature of the local site at the focused spot and generate a thermal transition. When the high intensity focused ultrasound is used for the skin, it uses the effect of removing wrinkles and restoring skin elasticity as degenerated tissues are regenerated by making a wound due to thermal change in the local site where fine ultrasound is focused.

The conventional high intensity focused ultrasound device is a transducer that generates ultrasound, and uses a method of transmitting strong ultrasound energy to a treatment site using a circular single-element ultrasound transducer. Since a plurality of focused spots should be formed to transmit ultrasound energy for skin treatment to a wide site, the conventional single-element ultrasound transducer adopts a method of mechanically moving a single-element ultrasound transducer to focus ultrasound on various body parts.

Therefore, the conventional high intensity focused ultrasound adopts a circular single-element ultrasound transducer, so, when a treatment depth (focused spot) is applied differently, there is a problem in that cartridges designed to have different ultrasound frequencies and geometrical structures of a transducer should be replaced each time. In order to solve this problem, there is the related art in which a lever, a motor, or a scanner is mounted on a handpiece tip to allow a user to arbitrarily select and adjust a depth of a target site manually, semi-automatically, or fully automatically so that ultrasound may be focused. However, the related art has a problem in that a mechanical movement is still required because the transducer should still be moved using a motor. As a result, the treatment time is prolonged and the position of the ultrasound transducer in the handpiece should be mechanically changed to change the treatment depth, so there is a limit to improving the treatment effect.

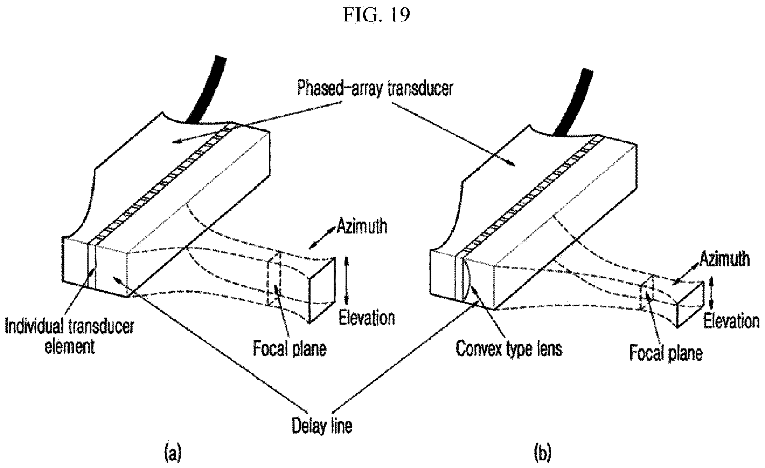

On the other hand, in the case of a diagnostic ultrasound device having low energy of generated ultrasound, an ultrasound transducer in the form of an array element is used as illustrated in FIGS. 19A and 19B. That is, by electrically controlling a transmission time of a plurality of ultrasound transducers in a lateral direction (x direction), ultrasound is focused on a desired location, and reflected ultrasound are received and imaged. In this case, in order to improve a diagnostic resolution, an elevation direction (y direction) may use a focusing technique to transmit high ultrasound energy to a focus. As the method, there are a method of using natural focusing as illustrated in FIG. 19A and a method of using acoustic lens focusing as illustrated in FIG. 19B.

Even in the case of the conventional diagnostic ultrasound devices, geometrical focusing may not be used in array element ultrasound transducers due to difficulties in the manufacturing process. As described above, the high intensity focused ultrasound device, which is an ultrasound device for treatment, uses a single ultrasound transducer element.

Despite these problems, it is difficult to implement a high intensity focused ultrasound device using an array type ultrasound transducer element. Since natural focusing is unsuitable due to energy dispersion, acoustic lens focusing or geometrical focusing is suitable for increasing a temperature of a lesion. However, in the case of the acoustic lens focusing, it is difficult to transmit high intensity energy to a treatment site due to the ultrasound attenuation caused by a material used as a lens. In addition, there is a problem in that the geometric focusing is difficult to form the geometric structure of the array type ultrasound transducers (at least dozens of ultrasound transducers having at least a focusable geometrical structure should be configured to control an electrical transmission time of the array type ultrasound transducers).

### [Disclosure]

### [Technical Problem]

The present disclosure attempts to provide a high intensity focused ultrasound probe and an ultrasound device in which a plurality of transducers capable of geometrical focusing are configured in an array form based on a geometrical structure.

The present disclosure attempts to provide a high intensity focused ultrasound probe and an ultrasound device in which a plurality of transducers can be easily manufactured at low cost, geometrically focused, and are configured in an array form.

The present disclosure attempts to provide a high intensity focused ultrasound probe and an ultrasound device in which a plurality of transducers capable of transmitting high energy to a treatment site while reducing grating lobes are configured in an array form.

The present disclosure attempts to provide a high intensity focused ultrasound probe and an ultrasound device in which a plurality of transducers capable of shortening a treatment time without mechanical movement by electrically controlling an ultrasound transmission time of each ultrasound transducer element are configured in an array form.

The present disclosure attempts to provide an ultrasound device in which ultrasound for treatment is focused on a plurality of spots or at a plurality of depths based on a plurality of transducers configured in an array form.

The present disclosure attempts to provide an ultrasound device that prevents a laceration of a patient due to ultrasound generated from a plurality of transducers configured in an array form.

### [Technical Solution]

An embodiment of the present disclosure provides a high intensity focused ultrasound probe and an ultrasound device.

According to an embodiment of the present invention, a high intensity focused ultrasound (HIFU) probe includes: a transducer module including a plurality of transducer elements generating ultrasound by receiving electrical signals; and a circuit providing a driving signal to the plurality of transducer elements based on a control signal received from a control system, in which the plurality of transducer elements having a concave shape in a first axial direction, which is a focusing direction of ultrasound, are arranged in an array in a second axial direction perpendicular to a first axis with a kerf interposed therebetween, and at least two different elements of the plurality of transducer elements are configured to emit high intensity focused ultrasound at different times based on the driving signal.

According to another embodiment of the present invention, a high intensity focused ultrasound (HIFU) device includes: a probe emitting high intensity focused ultrasound (HIFU); and a control system transmitting a control signal to the probe, in which the probe includes a transducer module including a plurality of transducer elements for generating ultrasound based on the control signal, the plurality of transducer elements having a concave shape in a first axial direction, which is a focusing direction of ultrasound, are arranged in an array in a second axial direction perpendicular to a first axis with a kerf interposed therebetween, and the control system controls at least two different elements among the plurality of transducer elements to generate the high intensity focused ultrasound at different times.

### [Advantageous Effects]

According to a high intensity focused ultrasound probe and an ultrasound device according to an embodiment of the present disclosure, it is possible to geometrically focus ultrasound generated based on a geometrical structure of a plurality of high intensity focused ultrasound transducer elements.

According to a high intensity focused ultrasound probe and an ultrasound device according to an embodiment of the present disclosure, a plurality of high intensity focused ultrasound transducer elements electrically controls a transmission time, thereby shortening a treatment time while improving a skin treatment effect inexpensively and easily.

According to a high intensity focused ultrasound probe and an ultrasound device according to an embodiment of the present disclosure, it is possible to prevent a laceration of a patient by differentiating cooling according to an operating mode of a plurality of high intensity focused ultrasound transducer elements.

### [Description of the Drawings]

FIG. 1 is a diagram for describing an implementation environment of an array type high intensity focused ultrasound probe and an ultrasound device according to an embodiment of the present disclosure.
FIG. 2 is a block diagram schematically illustrating a configuration of an array type high intensity focused ultrasound probe according to an embodiment of the present disclosure.
FIGS. 3A and 3B are perspective view and a plan view illustrating geometrical structures of transducer elements in the array type high intensity focused ultrasound probe according to the embodiment of the present disclosure.
FIGS. 4A and 4B is a perspective view and a side view for describing a part of a transducer module in the array type high intensity focused ultrasound probe according to the embodiment of the present disclosure.
FIG. 5 is a block diagram schematically illustrating a configuration of an array type high intensity focused ultrasound device according to an embodiment of the present disclosure.
FIG. 6 is a block diagram schematically illustrating a configuration of a transducer module of the array type high intensity focused ultrasound probe according to the embodiment of the present disclosure.
FIG. 7 is a diagram for describing a control signal transmitted to the array type high intensity focused ultrasound probe according to the embodiment of the present disclosure.
FIGS. 8 and 9 are diagrams illustrating an operating mode of the array type high intensity focused ultrasound probe according to the embodiment of the present disclosure.
FIGS. 10A to 10D are experiment results of a kerf interval of the array type high intensity focused ultrasound probe according to the embodiment of the present disclosure.
FIGS. 11A to 11D are experimental results of a device width of the array type high intensity focused ultrasound probe according to the embodiment of the present disclosure.
FIG. 12 is a perspective view illustrating a part of a transducer module in the array type high intensity focused ultrasound probe according to the embodiment of the present disclosure.
FIG. 13 is a flowchart for describing a method of manufacturing an array type transducer of an array type high intensity focused ultrasound probe according to an embodiment of the present disclosure.
FIG. 14 is a diagram for describing the method of manufacturing an array type transducer of an array type high intensity focused ultrasound probe according to the embodiment of the present disclosure.
FIG. 15 is a flowchart for describing the method of manufacturing an array type transducer of an array type high intensity focused ultrasound probe according to the embodiment of the present disclosure.
FIG. 16 is a diagram for describing the method of manufacturing an array type transducer of an array type high intensity focused ultrasound probe according to the embodiment of the present disclosure.
FIG. 17 is a flowchart for describing the method of manufacturing an array type transducer of an array type high intensity focused ultrasound probe according to the embodiment of the present disclosure.
FIG. 18 is a diagram for describing the method of manufacturing an array type transducer of an array type high intensity focused ultrasound probe according to the embodiment of the present disclosure.
FIGS. 19A and 19B are diagrams for describing an ultrasound focusing method of an ultrasound probe used in a conventional diagnostic ultrasound imaging device.

### [Mode for Invention]

Hereafter, embodiments of the present disclosure will be described in detail with reference to the accompanying drawings and the same or similar components are given the same reference numerals regardless of the numbers of figures and are not repeatedly described. The suffix "module" and/or "unit" for components used in the following description is given or mixed in consideration of only the ease of writing of the specification, and therefore, do not have meanings or roles that distinguish from each other in themselves. Further, when it is decided that a detailed description for the known art related to the present disclosure may obscure the gist of the present disclosure, the detailed description will be omitted. Further, it should be understood that the accompanying drawings are provided only in order to allow exemplary embodiments of the present disclosure to be easily understood, and the spirit of the present disclosure is not limited by the accompanying drawings, but includes all the modifications, equivalents, and substitutions included in the spirit and the scope of the present disclosure.

Terms including an ordinal number such as first, second, etc., may be used to describe various components, but the components are not limited to these terms. The terms are used only to distinguish one component from another component.

Further, in the present specification, it is to be understood that when one component is referred to as being "connected to" or "coupled to" another component, it may be connected or coupled directly to another component or be connected to another component with the other component interposed therebetween. On the other hand, it should be understood that when one element is referred to as being "connected directly to" or "coupled directly to" another element, it may be connected to or coupled to another element without the other element interposed therebetween.

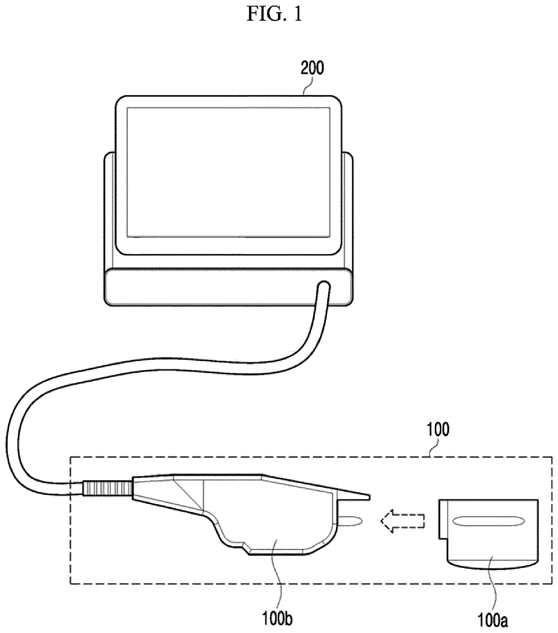

An ultrasound device according to an embodiment of the present disclosure includes an array type high intensity focused ultrasound probe 100 and a control system 200. An implementation environment of the array type high intensity focused ultrasound probe 100 and the control system 200 will be described with reference to FIG. 1.

Referring to FIG. 1, in the ultrasound device according to the embodiment of the present disclosure, the control system 200 controls the high intensity focused ultrasound probe 100 to focus ultrasound generated from the high intensity focused ultrasound probe 100 on a specific spot inside a body, thereby transmitting acoustic energy to internal tissues (dermis, superficial musculo-aponeurotic system (SMAS) layer, etc.) of a patient. The control system 200 may change a focused spot by electrically controlling transmission times of a plurality of array type transducer elements included in a transducer module of the high intensity focused ultrasound probe 100. The high intensity focused ultrasound probe 100 may transmit the high intensity focused ultrasound energy into the body for a non-invasive face lifting procedure, a skin tightening procedure, a non-invasive subcutaneous fat reduction or removal procedure, etc.

In one embodiment, the control system 200 controls a plurality of array type transducer elements of the high intensity focused ultrasound probe 100 so that ultrasound energy is focused at a plurality of locations at the same depth, or control the plurality of array type transducer elements of the high intensity focused ultrasound probe 100 so that the ultrasonic wave energy is focused at different depths.

The control system 200 may include a control unit including a treatment beamformer for calculating an ultrasound transmission time delay of each transducer element of the high intensity focused ultrasound probe 100 to change the focused spot.

In one embodiment, the control system 200 may include an input unit or an output unit for user input.

The input unit may include a user interface (UI) including a microphone and a touch interface for receiving information from a user, and the user interface may include mechanical and electronic interfaces or the like implemented in the device as well as a mouse and keyboard, and the method and form are not particularly limited as long as it is possible to input user instructions. The electronic interface includes a display capable of touch input. The output unit is for transmitting information to a user by expressing the output of the control system 200 to the outside, and may include a display, LED, speaker, etc., for expressing visual output, auditory output, or tactile output. When the high intensity focused ultrasound probe 100 includes an imaging transducer element module, the display may display an ultrasound image of an internal body tissue.

The control system 200 may include a peripheral device interface for data transmission to various types of connected external devices, and include a memory card port, an external device input/output (I/O) port, etc.

The control system 200 may control the high intensity focused ultrasound probe 100 by being connected to the high intensity focused ultrasound probe 100 by cable or wirelessly.

In one embodiment, the high intensity focused ultrasound probe 100 may include a handpiece (or may be called a wand) 100b and a cartridge 100a. In this case, the ultrasound transducer module may be implemented in the cartridge 100a. In another embodiment, the high intensity focused ultrasound probe 100 may be implemented by integrating the hand piece 100b and the cartridge 100a.

When the handpiece 100b and the cartridge 100a are implemented separately, electrical connection terminals and guide parts for physical coupling may be implemented in the handpiece 100b and the cartridge 100a so that the cartridge 100a can be coupled to the handpiece 100b. In one embodiment, the guide part may be implemented in a form such as a protruding bar, a protrusion, or the like in a direction in which the cartridge 100a is coupled to a front end portion of the handpiece 100b.

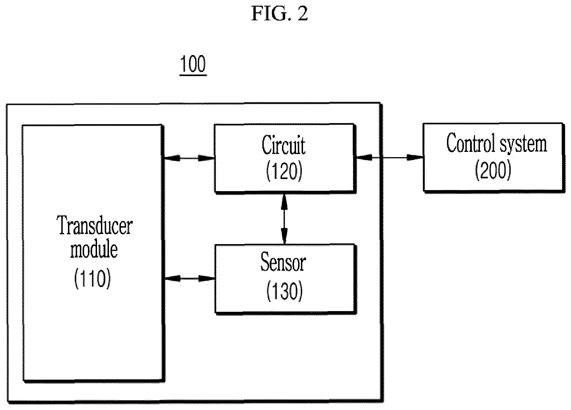

A configuration of the high intensity focused ultrasound probe 100 according to an embodiment of the present disclosure will be described with reference to FIG. 2.

The high intensity focused ultrasound probe 100 may include a transducer module 110 that generates ultrasound by receiving an electrical signal and a circuit 120 that inputs an electrical signal to the transducer module 110.

In one embodiment, when the high intensity focused ultrasound probe 100 is composed of the small number of arrays of transducer elements of the transducer module 110, the high intensity focused ultrasound probe 100 may include a stepping motor capable of one-dimensionally moving the transducer module 110 in order to focus the ultrasound on a plurality of focused spots by moving the handpiece 100b in the forward and backward directions (i.e., so that the ultrasound focused spot forms the plurality of spots at regular intervals along the same line), which is the axial direction of the handpiece 100b.

In one embodiment, the high intensity focused ultrasound probe 100 may include a sensor 130 capable of determining the position of the transducer module 110 may be included. The sensor 130 may be an optical sensor, a roller ball sensor, or the like.

As another embodiment, an encoder is connected to a motor for moving the transducer module 110 without using the sensor 130 to calculate the movement displacement and to determine the current position of the transducer module 110.

In one embodiment, as the sensor 130, sensors for various purposes that detect the movement of the transducer module 110 as well as the coupling between the handpiece 100b and the cartridge 100a (in this case, it may be a pressure sensor or a switch that detects electrical connection), detect an angle of the handpiece 100b with respect to the ground and a user's grip of the handpiece 100b, and the like, may be variously used.

In one embodiment, the sensor 130 may be a temperature sensor that measures at least one of a temperature of the transducer elements inside the transducer module 110, a temperature of degassed water in a loading space 112 in the transducer module 110, and a temperature of a patient's skin that contacts the high intensity focused ultrasound probe 100.

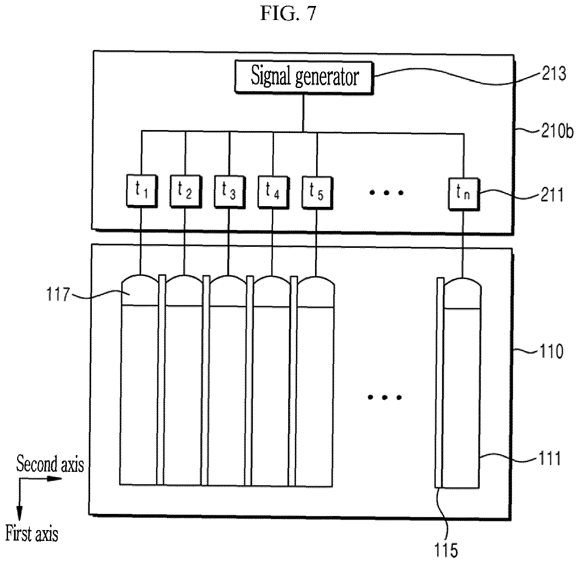

As another embodiment, when an array with a plurality of transducer elements is configured, the control system 200 may control at least some or all of the plurality of transducer elements based on a delay signal determined by a treatment beamformer 210 so that ultrasound is simultaneously or sequentially focused at a plurality of ultrasonic focusing positions. By varying the delay signal used to control the controlled transducer elements, the control system 200 may focus ultrasound on a plurality of focused spots in the forward and backward directions, which is the axial direction of the handpiece 100b, or focus ultrasound on different skin depths of a patient. In this case, the movement of the transducer module 110 may not be required. It will be described in detail with reference to FIGS. 7 to 9 below.

As another embodiment, when the array with the plurality of transducer elements is configured, the plurality of transducer elements (transducer modules) may be rotated at a predetermined angle around an axis in a longitudinal direction of the handpiece by using a motor. In this case, a wider area may be treated simultaneously.

As another example, in the one-dimensional arrangement of the transducer elements, an axis in a direction in which the transducer elements are coupled may be orthogonal to an axis in the longitudinal direction of the handpiece 100b.

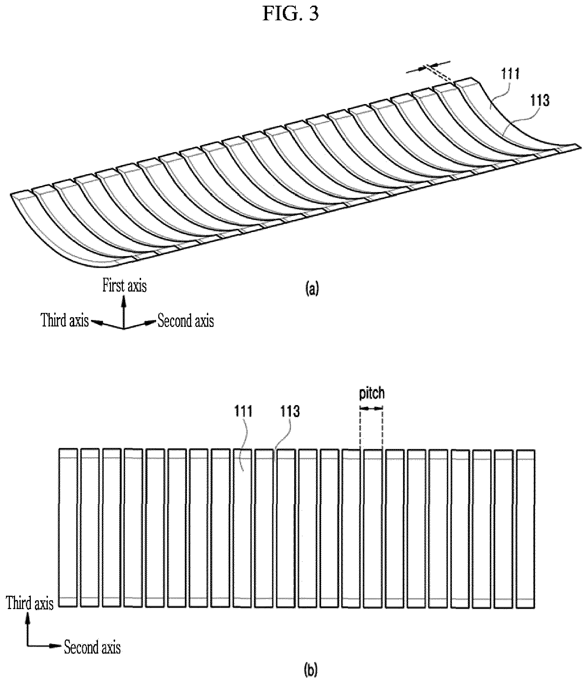

Therefore, in this case, the transducer module may be moved so that the plurality of array type transducer elements move along the axis in the longitudinal direction of the handpiece 100b (i.e., move along a third axial direction with reference to FIGS. 3A and 3B). In this case, the treatment time may be shortened by simultaneously transmitting ultrasound energy to a wider area.

The array type transducer elements included in the transducer module 110 of the array type high intensity focused ultrasound probe 100 according to the embodiment of the present disclosure will be described with reference to FIGS. 3A and 3B.

In this specification, an axis toward a spot where ultrasound is generated and focused from a plurality of transducer elements will be described as a first axis (height direction, also referred to as an axis in an axial direction), a direction in which the plurality of transducer elements are arranged will be described as a second axis (a lateral direction, also referred to as a lateral axis), and an axis perpendicular to the first and second axes while being the longitudinal direction in which each transducer element is extended will be described as a third axis (elevation direction axis, also referred to as elevation direction axis). The first axis, the second axis, and the third axis are orthogonal to each other. The expression "along an axis" includes heading in a direction similar to the axis while maintaining an angle with or without parallel with the axis.

The transducer elements of the array type high intensity focused ultrasound transducer module 110 according to the embodiment of the present disclosure may be a form in which the plurality of transducer elements 111 are arranged in the second axial direction in a one-dimensional array form with a kerf 113 interposed therebetween.

The kerf means an interval between the transducer elements, and is referred to as a device pitch, including widths of the kerf and the transducer element 111 in the second axial direction. A size of a grating lobe and a size of energy transmitted to an inside of a body may vary depending on the device pitch and the size of the kerf. Therefore, in the high intensity focused ultrasound transducer in the form of an array element, the optimal pitch of the ultrasound elements should be selected in advance so that a size of a main lobe increases and the size of the side lobe is minimized.

Each of the transducer elements 111 may have a concave geometrical shape in a direction opposite to the first axial direction, and this geometrical structure is related to setting a focused spot of ultrasound generated from the transducer elements 111. That is, an inner surface of each of the transducer elements 111 may have a concave shape in a direction opposite to the first axial direction based on a plane formed by the second axial direction and the third axial direction. The ultrasound generated by receiving electrical signals from each of the transducer elements 111 may travel in a first axial direction, and be focused at a specific depth spot inside the body to transmit energy.

Each of the transducer elements 111 may be elongated in the third axial direction. Accordingly, the length of each transducer element 111 in the third axial direction may be longer than the width in the second axial direction.

The ultrasound generated by each of the transducer elements 111 receiving an electrical signal travels in the first axial direction and are focused at a specific depth spot inside the body, so the transmitted energy (i.e., the ultrasound output energy of the transducer module) may be 0.5 J/cm² or more at the focused spot. Alternatively, the output energy per area of the transducer elements 111 may be 500 W/cm² or more. Accordingly, a thermal transition may be generated at the focused spot inside the body by transmitting energy so that the temperature of the focused spot inside the body is equal to or higher than a certain temperature.

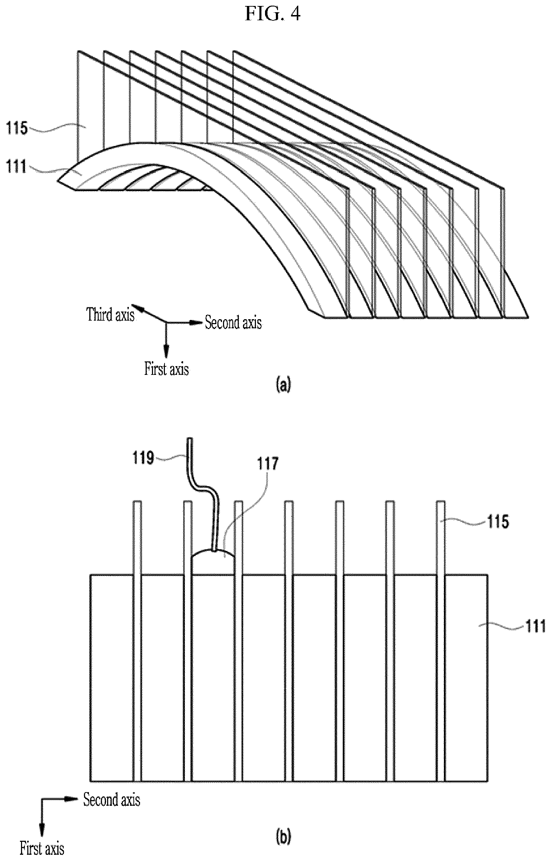

The array type transducer elements of the array type high intensity focused ultrasound transducer module 110 according to the embodiment of the present disclosure will be described with reference to FIG. 4.

The array type high intensity focused ultrasound transducer module 110 according to the embodiment of the present disclosure may include a separation member 115 arranged in the kerf 113 between the high intensity focused ultrasound transducer elements 111. The separation member 115 separates the plurality of transducer elements and prevents the plurality of transducer elements from contacting each other.

In one embodiment, the high intensity focused ultrasound transducer elements 111 are not directly connected to each other and arranged in an array form, but may be combined in a fixed form through the separation member 115 in a state of being separated from each other.

In one embodiment, the separation member 115 may be made of a material having a thermal stability of 300°C or higher, and may be made of, for example, polyimide (PI). Therefore, in order to prevent the separation member 115 from being thermally deformed when the signal line 119 is connected to the transducer element 111 by a soldering 117 method, the separation member 115 may be made of a material with little change in physical properties even when exposed to high temperatures for a long time.

In the plurality of transducer elements 111 of the array type high intensity focused ultrasound transducer module according to the embodiment of the present disclosure, a signal line for applying an electrical signal to a rear surface in a direction opposite to each ultrasound focusing direction may be connected by the soldering 117. Since the high intensity focused ultrasound transducer element applies a signal of high energy enough to cause a thermal transition to the desired lesion, it is possible to generate high heat instantaneously, and in order to connect the signal line to the transducer element 11 while preventing the separation of the signal line due to high heat, the soldering 117 method having a melting point of about 300°C or higher may be used.

In one embodiment, the length of the separation member 115 in the first axial direction may be longer than the length (thickness) of the transducer element 111 in the first axial direction. Accordingly, the separation member 115 may serve as a dam to prevent molten lead from being connected to other elements when the signal line is connected to the transducer element 111 by the soldering 117 method.

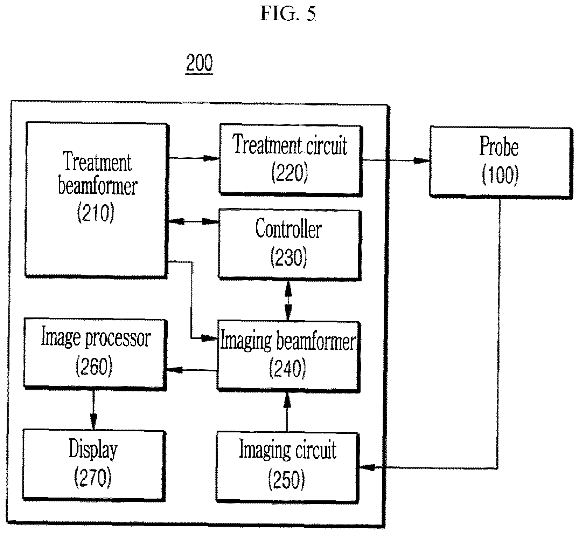

A configuration of a control system 200 for an ultrasound device according to an embodiment of the present disclosure will be described with reference to FIG. 5.

The control system 200 may include a treatment beamformer 210 that generates a delay signal so that at least some of the plurality of array type transducer elements of the ultrasound probe 100 receive electrical control signals at different times or generate ultrasound at different times.

In one embodiment, the delay signal generated by the treatment beamformer 210 may be transmitted to the probe 100 through the treatment circuit 220 including a pulser, a digital analog converter (DAC), and an amplifier. The treatment circuit 220 may include a digital device unit and an analog device unit. The plurality of delay signals may be transmitted to the pulser and converted into a high voltage signal (HV signal). The control system 200 may transmit, to the probe 100, a driving signal (HV signal) generated based on a delay signal calculating a point in time at which signals are input to each transducer element to control the position and depth of the focused spot of the ultrasound for treatment.

In one embodiment, the treatment circuit 220 may include a multiplexer, and transmit the driving signal to different transducer elements 111 of the probe 100 at different times according to the delay signal by the multiplexing.

In one embodiment, the control system 200 may generate an ultrasound B-mode image based on the received ultrasound in which the generated ultrasound (ultrasound for treatment or separate ultrasound for imaging) are reflected from the inside of the body. In this case, the probe 100 may include an analog front end circuit for processing an electrical signal (referred to as a received ultrasound signal) obtained by converting an ultrasound signal. The analog front end circuit may include a low-noise amplifier (LNA) and depth gain compensation (TGC) for attenuation correction. The ultrasound received by the transducer elements 111 in the probe 100 are converted into electrical signals and transmitted to the imaging circuit 250, and the imaging circuit 250 may include an analog digital converter (ADC), a first-input-first-output (FIFO) memory, etc. The digitally processed received ultrasound signal may be subjected to Rx focusing based on the delay signal of the treatment beamformer 2110 in the imaging beamformer 240, subjected to scan converting in the image processor 260, and displayed on a display 270.

In one embodiment, the control system 200 may include a controller 230 that controls whether to operate in an ultrasound imaging mode (B mode) for imaging the received ultrasound signal or a treatment mode for performing the high intensity focused ultrasound treatment on an object.

In one embodiment, the control system 200 or a separate external device may include a motor that circulates degassed water into the loading space 112 in the transducer module 110, and a cooling device that cools the degassed water discharged from the loading space 112. The controller 230 may control the circulation speed of the degassed water introduced into or discharged from the loading space 112 based on at last any one of the temperature of the transducer elements inside the transducer module 110 measured by the temperature sensor 130, the temperature of the degassed water of the loading space 112 in the transducer module 110, and the temperature of the patient's skin in contact with the high intensity focused ultrasound probe 100. Therefore, it is possible to prevent a laceration of a patient due to the high intensity focused ultrasound treatment.

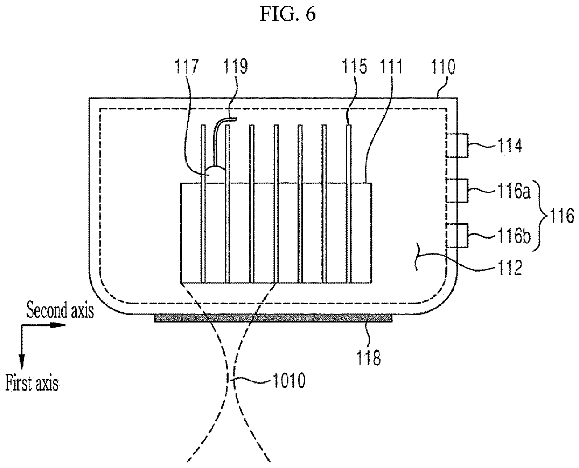

A conceptual configuration of the transducer module 110 of the array type high intensity focused ultrasound probe 100 according to the embodiment of the present disclosure will be described with reference to FIG. 6. For convenience of description, FIG. 6 illustrates a state in which the transducer module 110 is implemented as the cartridge 100a.

In one embodiment, the transducer module 110 may be implemented as a separate housing so as to be detachable from the handpiece 100b. In another embodiment, the transducer module 110 may be implemented in the same housing as the handpiece 100b. The embodiment described with reference to FIG. 6 will be described as an embodiment in which the transducer module 110 is implemented as a separate housing from the handpiece 100b.

The transducer module 110 includes the array type transducer elements 111 in which the plurality of transducer elements 111 are arranged with a kerf interposed therebetween. In one embodiment, the separation member 115 for preventing the contact between the transducer elements 111 may be included in the kerf between the transducer elements 111.

In one embodiment, in the transducer module 110, two axes, a longitudinal axis of the handpiece 100b and an arrangement direction axis in which the plurality of transducer elements are arranged are parallel to each other, but in another embodiment, may intersect each other.

In one embodiment, the transducer module 110 may include the loading space 112 loaded with the degassed water or other materials having high acoustic transparency in an internal space, and the loading space 112 may be filled with the degassed water. In another embodiment, the loading space 112 may be maintained as an empty space without being filled with a separate material.

In one embodiment, the transducer module 110 may include a circulation passage 116 capable of circulating the degassed water in the loading space 112, and the circulation passage 116 may include an inlet 116a and an outlet 116b. The degassed water cooled from the outside of the transducer module 110 is introduced into the loading space 112 inside the transducer module 110 through the inlet 116a, and the degassed water whose temperature rises due to the heat generated by the transducer elements 111 may be discharged from the loading space 112 to the outside through the outlet 116b.

In one embodiment, when the transducer module 110 is implemented as a separate housing detachably from the handpiece 100b, the transducer module 110 may include an electrical interface 114 that may be electrically connected to the control system 200 through the circuit of the handpiece 100b. The electrical interface 114 may include a PCB that serves as an electrical/mechanical connector.

In one embodiment, the housing of the transducer module 110 may have an open window, through which ultrasound can pass, on one axis in a direction in which ultrasound generated from the transducer elements 111 is radiated, and when the loading space 112 is filled with a material having high acoustic transparency, such as degassed water, a cover member 118 may be formed to prevent the material from escaping through the window.

The ultrasound generated from the plurality of transducer elements 111 based on different delays are focused on a specific spot 1010 in one axial direction.

A control method based on a delay signal of a plurality of array type ultrasound transducer elements 111 according to another embodiment of the present disclosure will be described with reference to FIG. 7. FIG. 7 is described on the premise that the delay element 211 is separately connected to each of the transducer elements 111, but in another embodiment, an analog front end portion 210b may include a high voltage multiplexer (HV multiplexer). In this case, the delay element 211 is not connected to each of the transducer elements 111, and an element to be activated (to generate ultrasound) may be selected from among the plurality of transducer elements 111 through the high voltage multiplexer. Also, the analog front end portion 210b may be implemented inside the control system 200 or inside the handpiece 100b.

Referring to FIG. 7, the delay signal may be transmitted to the analog front end portion 210b, and the analog beamforming may be performed in the analog front end portion 210b to control at least two different elements among the plurality of transducer elements 111 to generate the high intensity focused ultrasound at different times based on the driving signal of the control system 200. In one embodiment, the analog front end portion 210b may include a signal generator 213, a delay element 211, a high voltage amplifier, a filter (DAC filter, etc.), and an impedance matching transformer. The RF pulse generated by the signal generator 213 is transmitted to the delay element 211, and the delay element 211 may supply a driving signal to the plurality of transducer elements 111 according to a delay value referring to a delay table stored in a separate memory.

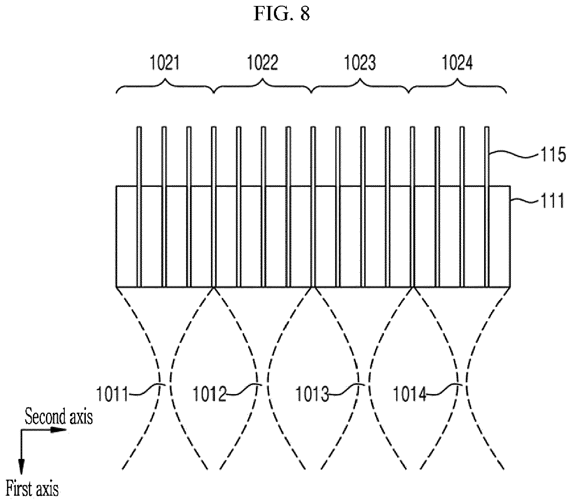

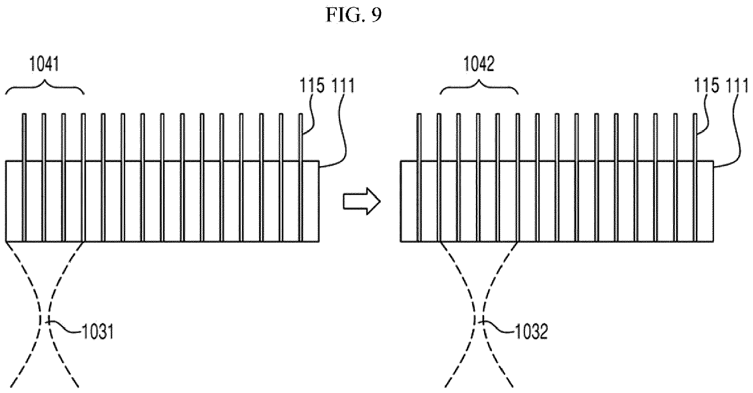

Different modes of generating the high intensity focused ultrasound of the array type transducer elements 111 according to the embodiment of the present disclosure will be described with reference to FIGS. 8 and 9.

Referring to FIG. 8, the plurality of transducer elements 111 may be exclusively divided and belong to a plurality of different subgroups 1021, 1022, 1023, and 1024. Being divided exclusively means that any one transducer element belongs to only any one subgroup.

In one embodiment, each subgroup 1021, 1022, 1023, and 1024 of the plurality of transducer elements 111 has focused spots 1011, 1012, 1013, and 1014 having the same depth but at different positions for each subgroup 1021, 1022, 1023, and 1024. Accordingly, the delays transmitted to the subgroups 1021, 1022, 1023, and 1024 may be equal to each other.

In one embodiment, the control system 200 may control so that each subgroup 1021, 1022, 1023, and 1024 of the plurality of transducer elements 111 simultaneously focuses the high intensity focused ultrasound on the plurality of focused spots 1011, 1012, 1013, and 1014. Accordingly, the high intensity focused ultrasound treatment can be simultaneously performed on a plurality of locations inside the patient's tissue, thereby shortening the patient's treatment time.

Referring to FIG. 9, the plurality of transducer elements 111 may be mutually divided and belong to a plurality of different subgroups 1041 and 1042. Being divided mutually means that any one transducer element does not belong to any one subgroup 1041 but belongs to another subgroup 1042.

In one embodiment, each subgroup 1041 and 1042 of the plurality of transducer elements 111 may have the focused spots 1031 and 1032 having the same depth but at different positions for each subgroup 1041 and 1042. Accordingly, the form of the delays transmitted to each subgroup 1041 and 1042 may be equal to each other.

In one embodiment, the control system 200 may control so that each subgroup 1041 and 1042 of the plurality of transducer elements 111 focuses the high intensity focused ultrasound on the plurality of focused spots 1031 and 1032 at different times. Therefore, the high intensity focused ultrasound treatment may be performed on the plurality of locations inside the patient's tissue without mechanically moving the transducer elements, thereby shortening the patient's treatment time.
In one embodiment, the control system 200 may control the plurality of transducer elements 111 can be operated by being divided into in a first mode in which the plurality of transducer elements 111 are exclusively divided into a plurality of different subgroups and are controlled and a second mode in which the plurality of transducer elements are mutually divided into the plurality of different subgroups and controlled, and control the plurality of transducer elements 111 in the corresponding mode according to an operator's selection.

In one embodiment, the control system 200 may control the plurality of transducer elements 111 so that a pitch between the spots where the high intensity focused ultrasound is focused in the first mode is wider than a pitch between spots where the high intensity focused ultrasound is focused in the second mode.

Therefore, the high intensity focused ultrasound treatment may be performed in a different manner according to the condition of the patient.

In one embodiment, the control system 200 may control the plurality of transducer elements 111 so that the energy (i.e., the ultrasound output energy of the transducer module) transmitted to the focused spot is different in the first mode and the second mode. For example, the plurality of transducer elements 111 may be controlled so that the energy transmitted to the focused spot is lower in the second mode in which the pitch between the spots on which the high intensity focused ultrasound is focused is denser.

In one embodiment, the control system 200 may control the operating speed of the circulation motor that circulates the degassed water so that the circulation speed of the degassed water in the loading space 112 inside the transducer module 110 is different in the first mode and the second mode, or differently control the cooling rate for cooling the degassed water.

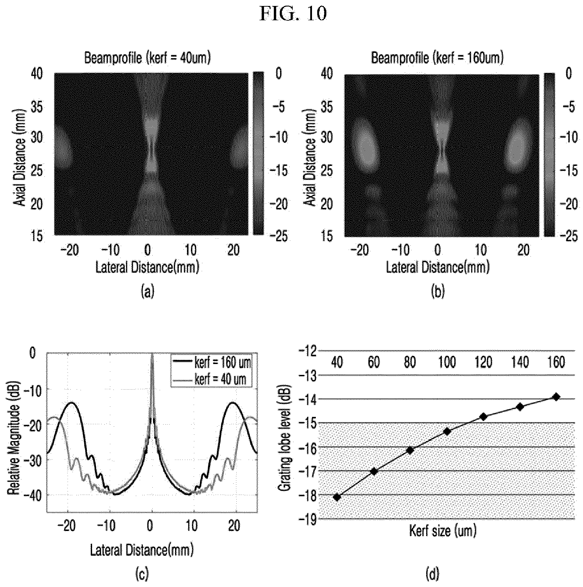

The kerf width (size) of the array type high intensity focused ultrasound transducer module and the width of the transducer element according to the embodiment of the present disclosure will be described with reference to FIGS. 10A to 10D.

FIGS. 10A to 10D illustrate simulation results for the side lobe size according to the kerf width. In the simulation, the width of the transducer element was fixed at about 460 µm, and the simulation was performed at intervals of 20 µm with the kerf width from 20 µm to 160 µm. In this case, the size of the side lobe was set within the range of the kerf width less than about -15 dB compared to the maximum energy.

FIG. 10A illustrates a beam profile when the kerf width is 40 µm, and FIG. 10B illustrates the beam profile when the kerf width is 160 µm. FIG. 10C illustrates a line plot when the kerf width is 40 µm and 160 µm. FIG. 10D illustrates a relative size of the side lobe according to the change in the kerf width as a graph. When the size of the kerf width is 100 µm, it can be seen that the relative size of the side lobe becomes about -15.34 dB, and when the size of the kerf width is 120 µm, it can be seen that the relative size of the side lobe becomes -14.74 dB, which is larger than -15 dB. Accordingly, it can be seen that the kerf width may increase to about 100 µm under the corresponding conditions.

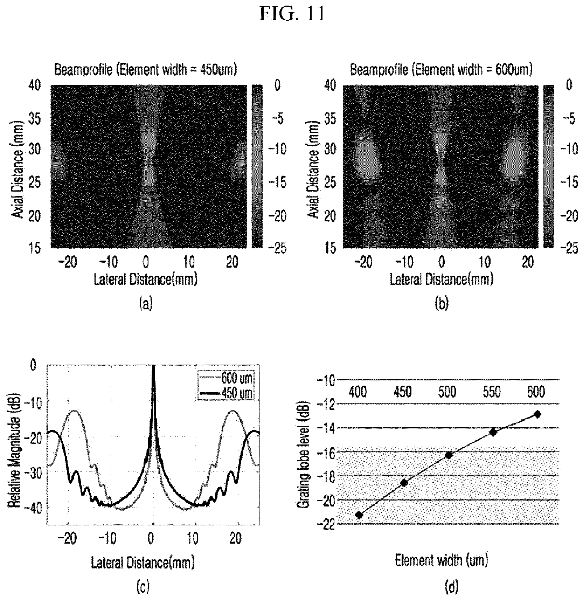

Contrary to FIGS. 10A to 10D, in FIGS. 11A to 11D, the kerf width was fixed at 40 µm, and the simulation was performed while changing the device width from 400 µm to 600 µm at intervals of 50 µm.

In this way, the range of the device width was set so that the relative size of the side lobe was less than about -15dB. FIG. 11A illustrates the beam profile when the device width is 450 µm and FIG. 11B illustrates the beam profile when the device width is 600 µm. FIG. 11C illustrates the line plot when the device widths are 450 µm and 600 µm. FIG. 11D illustrates the relative size of the side lobe according to the change in the device width as a graph. When the size of the device width is 500 µm, it can be seen that the relative size of the side lobe is about -16.26 dB, and when the size of the device width is 550 µm, the relative size of the side lobe is -14.35 dB, which is larger than -15 dB, and thus, it can be seen that the device width may increase up to 500 to 550 µm under the corresponding conditions.

The simulations performed in FIGS. 10 and 11 are simulation results performed when the device width and the kerf width are fixed and a single frequency is 4 MHz, and the set range may vary according to various conditions. In FIGS. 10 and 11, since the size of the side lobe increases as the widths of the device and the kerf increase, the total energy may decrease. As a result, the device width, the kerf width, and the device pitch may be determined so that the total energy may be maximized.

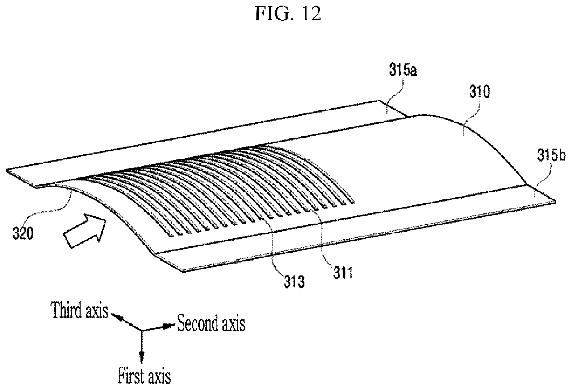

Another embodiment of the array type high intensity focused ultrasound probe according to the embodiment of the present disclosure will be described with reference to FIG. 12.

Detailed descriptions of parts overlapping with those described above will be omitted and will be described focusing on the transducer module.

The high intensity focused ultrasound probe 100 may include a transducer module. In the transducer module, the transducer element 311, which is a part that generates ultrasound, may be formed in a plurality of parts of curved transducer units 310 and 320 due to a kerf 313.

The transducer module may include the curved transducer parts 310 and 320 and bent parts 315a and 315b that are bent in a direction opposite to the ultrasound focusing direction on at least one of both ends of the transducer parts 310 and 320 in a third axial direction to form a flat surface. The curved transducer units 310 and 320 may have a concave shape in a direction opposite to a first axial direction, which is the focusing direction of ultrasound.

In one embodiment, a portion of the curved transducer parts 310 and 320 may be separated due to the kerf 313 to apply an electrical signal to the plurality of transducer elements 311 formed in a plurality of parts to generate ultrasound, and a signal line for applying an electrical signal may be connected to the transducer element 311 by the soldering 117 at the rear surface 310 opposite to the ultrasound focusing direction. In one embodiment, the separation member as illustrated in FIGS. 4A and 4B may be arranged on the kerf 313.

In one embodiment, a ground electrode may be formed in at least a portion of the front surface 320 in the ultrasound focusing direction in the curved transducer unit, and may be formed by a sputtering method to reduce the ultrasound attenuation. The ground electrode may be formed in at least a portion of the front surface 320 of the plurality of transducer elements 311 in the ultrasound focusing direction.

The transducer elements 311 of the plurality of parts formed in a plurality of parts by separating a portion of the transducer parts 310 and 320 mean the parts separated by the kerf 313 formed by extending along the third axial direction, accordingly, at least one end of both ends of the transducer elements 311 in the third axial direction may be connected to each other.

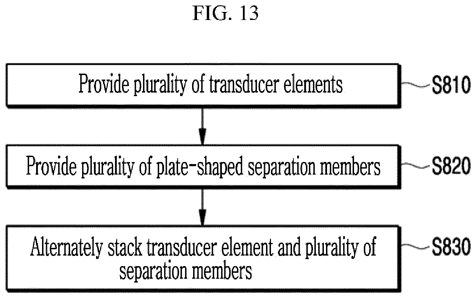

A method of manufacturing an array type high intensity focused ultrasound probe according to an embodiment of the present disclosure will be described with reference to FIGS. 13 to 18.

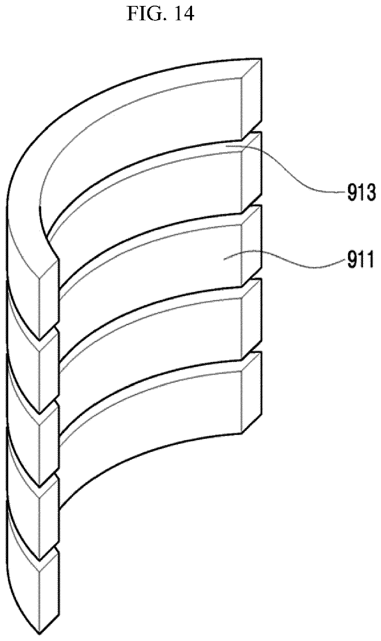

First, a method of manufacturing an array type transducer of a high intensity focused ultrasound probe in a stacked manner will be described with reference to FIGS. 13 and 14.

When an electrical signal is applied, a plurality of transducer elements 911 that have a predetermined thickness in the first axial direction and a predetermined concave curvature in the direction opposite to the first axial direction, which is a direction in which ultrasound is generated and focused and have a shape extended in the third axial direction perpendicular to the first axis is provided (S810).

A plurality of plate-shaped separation members having a predetermined second thickness are provided (S820), and the plate-shaped separation member may be made of a material having a thermal stability of 300°C or higher, and may be, for example, polyimide.

The transducer module is manufactured so that a plurality of curvature-type transducer elements and a plurality of plate-shaped separation members are alternately stacked in the first axial direction and in the second axial direction orthogonal to the third axis (S830) to arrange the plate-shaped separation members on the kerf 913. Thereafter, the signal lines may be connected to the rear surface (surface opposite to the ultrasonic focusing direction) of the plurality of curvature-type transducer elements using the soldering 117 method, and a ground electrode may be formed on the front surface. The stacking method has an excellent effect of ease of manufacture.

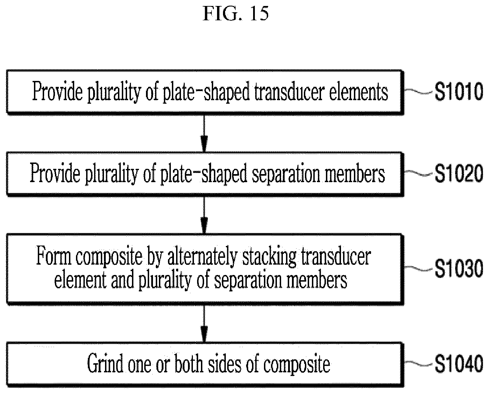

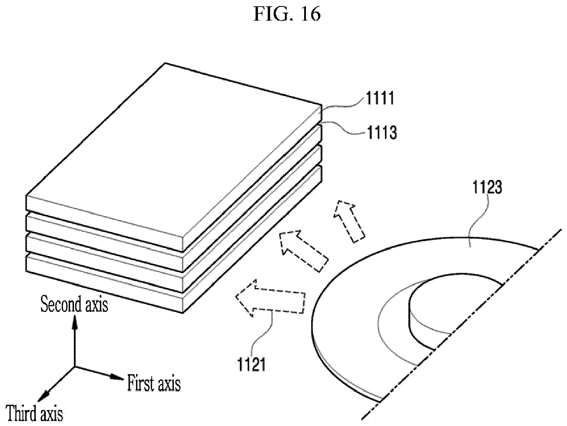

A method of manufacturing an array type transducer of a high intensity focused ultrasound probe by a polishing or grinding method will be described with reference to FIGS. 15 and 16.

A plurality of plate-shaped transducer elements 1111 having a predetermined thickness in a second axial direction are provided (S1010), and a plurality of plate-shaped separation members 1113 having different predetermined thicknesses are provided (S1020). Thereafter, the plurality of transducer elements 1111 and the plurality of separation members 1113 are alternately stacked in the second axial direction to form a composite (S1030), and one or both sides of the composite that are perpendicular to the first axis are ground (1121) by a grinding machine 1123 (S1040). In this case, a curvature of a grinding blade of the grinding machine 1123 is set to have a geometrical structure considering the ultrasound focused spot. One surface is ground to have a predetermined thickness concave in the first axial direction while having a predetermined thickness in the first axial direction, which is the direction in which ultrasound are generated and focused, and the rear surface (surface on an opposite side to the ultrasound focusing direction) which is the other surface in the opposite direction may be ground to have a predetermined convex curvature in the first axial direction. The grinding method has an effect of excellent ease of manufacturing and low sensitivity.

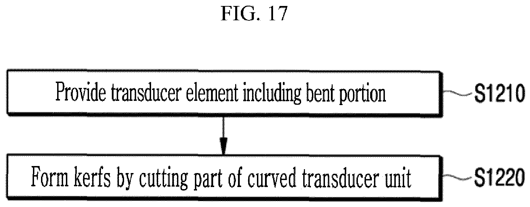

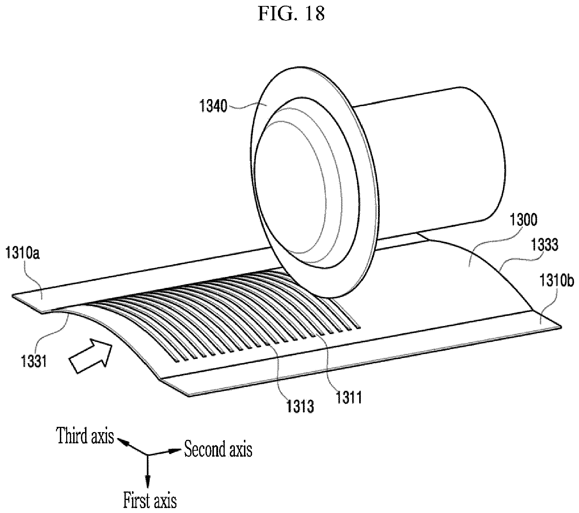

A method of manufacturing an array type transducer of a high intensity focused ultrasound probe using a cutting method will be described with reference to FIGS. 17 and 18.

A transducer module 1300 that includes curved transducer units 1331 and 1333 having partially a concave shape in the first axial direction, which is the ultrasound focusing direction, and curved parts 1310a and 1310b formed by being bent in the opposite direction to the ultrasound focusing direction at both ends in the third axial direction perpendicular to the first axis of the transducer units 1331 and 1333 is provided (S1210).

A plurality of kerfs 1313 extending in a third axial direction from the curved transducer units 1331 and 1333 and extending in a second axial direction perpendicular to the first and third axes are formed by cutting at least a portion of the curved transducer units 1331 and 1333 in the second axial direction perpendicular to the first axis and the third axis through a cutting machine 1340 (S1220).

A portion of the curved transducer parts 1331 and 1333 may be separated due to the kerf 1313 to apply an electrical signal to the plurality of transducer elements 1311 formed at a plurality of parts to generate ultrasound, and a signal line for applying an electrical signal may be connected to the transducer element 1311 by the soldering 117 at the rear surface 1333 opposite to the ultrasound focusing direction. In one embodiment, the separation member as illustrated in FIGS. 4A and 4B may be arranged on the kerf 1313. In one embodiment, the ground electrode may be formed on the front surface 1331 of the curved transducer units 1331 and 1333 by the sputtering method.

In the specification of the present disclosure (particularly in the claims), the use of the term "above" and similar indicating terms may correspond to both singular and plural. In addition, when the range is described in the present disclosure, as including the invention to which individual values belonging to the range are applied (unless otherwise stated), each individual value constituting the range is described in the detailed description of the invention.

Unless an order is explicitly stated or stated to the contrary for steps constituting the method according to the present disclosure, the steps may be performed in any suitable order.

The present disclosure is not necessarily limited to the order of description of the steps. The use of all examples or exemplary terms (e.g., etc.) in this disclosure is simply to explain the present disclosure in detail, and the range of the present disclosure is limited due to the examples or exemplary terms unless limited by the claims. In addition, those skilled in the art can appreciate that various modifications, combinations and changes can be made according to design conditions and factors within the scope of the appended claims or equivalents thereof.

Therefore, the spirit of the present disclosure should not be limited to these exemplary embodiments, but the claims and all of modifications equal or equivalent to the claims are intended to fall within the scope and spirit of the present disclosure.

## Claims

1. A high intensity focused ultrasound (HIFU) probe, comprising:
a transducer module including a plurality of transducer elements generating ultrasound by receiving electrical signals; and
a circuit providing a driving signal to the plurality of transducer elements based on a control signal received from a control system,
wherein the plurality of transducer elements having a concave shape in a direction opposite to a first axial direction, which is a focusing direction of ultrasound, are arranged in an array in a second axial direction perpendicular to a first axis with a kerf interposed therebetween, and
at least two different elements of the plurality of transducer elements are configured to emit high intensity focused ultrasound at different times based on the driving signal.

2. The HIFU probe of claim 1, wherein:
each of the plurality of transducer elements is formed in a geometrical structure that causes ultrasound generated by receiving an electrical signal to be focused on a predetermined spot in the first axial direction, and each of the plurality of transducer elements has a shape in which a length in a third axial direction perpendicular to the first axial direction and the second axial direction is greater than a length in the second axial direction.

3. The HIFU probe of claim 1, wherein:
ultrasonic output energy of the transducer module is 0.5 J/cm² or more at a focused spot.

4. The HIFU probe of claim 1, wherein:
a length of a separation member arranged on the kerf in the first axial direction is greater than the length of the transducer element in the first axial direction.

5. The HIFU probe of claim 4, further comprising:
a signal line connected to rear surfaces of each of the plurality of transducer elements in a direction opposite to an ultrasound focusing direction by soldering to apply an electrical signal.

6. The HIFU probe of claim 1, further comprising:
a separation member arranged on the kerf and made of a material having a thermal stability of 300°C or higher.

7. The HIFU probe of claim 6, wherein:
the separation member is made of polyimide.

8. The HIFU probe of claim 1, wherein:
each of the plurality of transducer elements is not directly connected in the second axial direction.

9. The HIFU probe of claim 1, wherein:
at least one end of both ends of each of the plurality of transducer elements in the third axial direction is connected to each other.

10. The HIFU probe of claim 9, wherein:
a ground electrode is formed in at least a portion of a front surface of each of the plurality of transducer elements in an ultrasound focusing direction.

11. The HIFU probe of claim 10, wherein:
an extension part of at least one end of both ends of each of the plurality of transducer elements in the third axial direction includes a bent part bent in a direction opposite to the ultrasound focusing direction.

12. The HIFU probe of claim 1, wherein:
the transducer module includes a loading space in which degassed water is loaded, and
the transducer module and a first housing in which the loading space is arranged are detachably connected to a second housing in which the circuit is arranged, and the loading space is connected to an inlet tube or an outlet tube capable of circulating the degassed water.

13. A high intensity focused ultrasound (HIFU) device, comprising:
a probe emitting high intensity focused ultrasound (HIFU); anda control system transmitting a control signal to the probe,
wherein the probe includes a transducer module including a plurality of transducer elements for generating ultrasound based on the control signal,
the plurality of transducer elements having a concave shape in a first axial direction, which is a focusing direction of ultrasound, are arranged in an array in a second axial direction perpendicular to a first axis with a kerf interposed therebetween, and
the control system controls at least two different elements among the plurality of transducer elements to generate the high intensity focused ultrasound at different times.

14. The HIFU device of claim 13, wherein:
each of the plurality of transducer elements is formed in a geometrical structure that causes ultrasound generated base on the control signal to be focused on a predetermined spot in the first axial direction, and each of the plurality of transducer elements has a shape in which a length in a third axial direction perpendicular to the first axial direction and the second axial direction is longer than a length in the second axial direction.

15. The HIFU device of claim 13, wherein:
the probe further includes a loading space in which degassed water is loaded,
The probe further includes a temperature sensor for measuring a temperature of at least one of the transducer elements, the degassed water in the loading space, and an object in contact with the probe, and
the control system controls an operating speed of a circulation motor connected to the loading space to circulate the degassed water or a cooling rate to cool the degassed water based on the temperature measured by the temperature sensor.

16. The HIFU device of claim 13, wherein:
the plurality of transducer elements are exclusively divided and belong to a plurality of different subgroups, and
the control system transmits the control signal to the probe so that the high intensity focused ultrasound emitted by the transducer elements of the subgroup are simultaneously focused at different spots.

17. The HIFU device of claim 13, wherein:
at least some of the plurality of transducer elements are mutually divided and belong to a plurality of different subgroups, and
the control system transmits the control signal to the probe so that the high intensity focused ultrasound emitted by the transducer elements of the subgroup are focused at different spots at different times.

18. The HIFU device of claim 13, wherein:
the control system is operated
in a first mode in which the plurality of transducer elements are is exclusively divided into a plurality of different subgroups and are controlled, and
a second mode in which the plurality of transducer elements are mutually divided into the plurality of different subgroups and controlled, and
the control system transmits the control signal to the probe in the first mode and the second mode so that a pitch between spots on which the high intensity focused ultrasound is focused in the first mode is wider than a pitch between spots on which the high-intensity focused ultrasound is focused in the second mode.

19. The HIFU device of claim 13, wherein:
the probe further includes a loading space in which degassed water is loaded, and
the control system is operated
in a first mode in which the plurality of transducer elements are exclusively divided into a plurality of different subgroups and are controlled,
a second mode in which the plurality of transducer elements are mutually divided into the plurality of different subgroups and controlled, and
the control system controls an operating speed of a circulation motor connected to the loading space to circulate the degassed water or a cooling rate for cooling the degassed water differently in the first mode and the second mode.
